# EUROPEAN PATENT SPECIFICATION

(11) **EP 1 911 577 B1**
(45) Date of publication and mention of the grant of the patent: **07.03.2012**
(21) Application number: 07111962.2
(22) Date of filing: 06.07.2007
(51) Int. Cl.: B32B 3/30, A61F 13/15, B29D 7/01, B29C 59/06

(54) **Dry top formed film**
Trockene, von oben geformte Folie
Film formé à sec

(30) Priority: 13.10.2006 US 549265
(43) Date of publication of application: 16.04.2008
(73) Proprietor: Tredegar Film Products Corporation, Richmond, VA 23225 (US)
(72) Inventor: Seyler, Rickey J., Chesterfield, VA 23828 (US); Thomas, Paul E., Terre Haute, IN 47802 (US)
(74) Representative: von Kreisler Selting Werner

(56) References cited:
- EP-A- 1 184 015
- WO-A-2004/096472
- US-A- 5 622 584

## Description

### FIELD OF INVENTION

The present invention relates, generally, to an apertured film for use in an absorbent article as a topsheet. More specifically, the invention relates to an apertured film with raised hydrophobic lanes which impart a dry feel to the wearer.

### BACKGROUND OF INVENTION

A variety of absorbent articles adapted to absorb body fluids are well known. Examples of such absorbent articles include diapers, incontinent articles, and sanitary napkins.

One problem associated with absorbent articles is maintaining the dryness of the skin-contacting surface of the article. Generally, when the skin-contacting surface is drier, the absorbent article is more comfortable. Attempts have been made to reduce surface wetness in disposable diaper structures. One common approach is interposing a perforated thermoplastic film or a nonwoven layer between a topsheet and the absorbent core of the absorbent article to prevent rewet of the topsheet. This method, however, is faced with a number of drawbacks. In particular, the method employs a topsheet that is typically a nonwoven material with a large surface area that comes in contact with the wearer of the absorbent article. The topsheet does not provide sufficient air space between the topsheet and the wearer for helping to maintain a dry skin-contacting surface. Moreover, non-woven material exhibits capillary action by wicking moisture toward the absorbent core of an absorbent article. This capillary action, however, can also wick moisture back toward the skin-contacting surface particularly if there is moisture that does not penetrate the perforated thermoplastic film that sits below the non-woven material. Another problem is that the fibers of the non-woven material can trap moisture close to the skin-contacting surface of the non-woven material. This leads to a feeling of wetness for the wearer.

Other approaches for improving the dryness of the skin-contacting surface of an absorbent article include using an apertured formed film as a topsheet. A problem with this approach is that there is a considerable upper surface area which does not allow passage of fluid through the film to an absorbent core below. This upper surface can remain wet particularly if this surface is hydrophilic. This wetness can cause the areas of film between the apertures to adhere to the wearer's skin. Another problem with this approach is that some consumers do not like the plastic feel associated with formed films.

Another problem with typical absorbent articles is caused by repeated insults to the article. Upon repeated insults, an undesirable leakage or undesirable feeling of wetness by the wearer may occur due to the absorbent core material of the absorbent article becoming saturated in the repeat insult region. As a result, the additional fluid may be unabsorbed and remain on the body-facing side of the absorbent article or the unabsorbed fluid may flow laterally over or through the topsheet from an area of saturated core material to an area of unsaturated core material for absorption. This causes a highly uncomfortable and undesirable sensation.

Therefore, there is a need for a topsheet that maintains a dry skin-contacting surface and that allows for the efficient channeling of fluids from an area adjacent a saturated portion of the core to an area adjacent an unsaturated portion of the core. The present invention fulfills these needs among others.

### SUMMARY OF THE INVENTION

The present invention relates to an apertured film which is suitable for a number of different applications, including, for example, as a topsheet for an absorbent article.

With respect to absorbent articles a topsheet is needed that has good fluid acquisition while also providing a dry skin-contacting surface with an improved feel. To address these issues, applicants have developed an apertured film with skin-contacting raised lanes that are hydrophobic to impart a dry feel to the wearer and an apertured continuous layer at a lower surface that is hydrophilic to provide good fluid acquisition. As an added benefit, when this apertured film is used as a topsheet in an absorbent article, which further comprises an absorbent core and a backsheet, the raised lanes alone or in conjunction with raised ridges in the apertured film act to redirect fluid from an area adjacent to saturated absorbent core to an area adjacent to unsaturated absorbent core.

Accordingly, one embodiment of the invention is an apertured film for use as a topsheet for an absorbent article. The apertured film comprises an apertured continuous layer having a male side and an opposite female side, the female side having a surface that is hydrophilic; and raised hydrophobic lanes extending upward from the female side of the apertured continuous layer. The raised hydrophobic lanes contact the skin of the wearer and impart a dry feel and also present a smaller skin-contacting surface allowing more efficient air flow between the absorbent article and the wearer's skin. The hydrophilic female side of the apertured continuous layer improves the fluid acquisition of the topsheet.

With respect to redirecting unabsorbed fluid, the raised lanes, particularly when they are substantially parallel to each other, act to redirect unabsorbed fluid to areas adjacent to unsaturated core. Moreover, applicants have also found that raised ridges in the apertured continuous layer help to channel unabsorbed fluid to areas of unsaturated core. Accordingly, in a preferred embodiment, the apertured continuous layer also has raised ridges extending upward from the female side in registration with the raised lanes.

In order to have good fluid acquisition, the female side or body-facing side of the apertured continuous layer is hydrophilic. Accordingly, one aspect of the invention is treating the female side of the apertured continuous layer to be hydrophilic. In a preferred embodiment, the apertured continuous layer contains a non-migrating surfactant, preferably an oligomeric ethoxylate.

With respect to the plastic feel of the film, microstructures may be formed on the upper surface of the raised lanes to impart a cloth like feel to the apertured film.

The present invention also relates to a method for making an apertured film for use as a topsheet for an absorbent article. Accordingly, one embodiment of the invention is a method for making an apertured film for use as a topsheet for an absorbent article, comprising (a) extruding a flat continuous layer of a polymer melt having an upper and lower surface onto a screen assembly; (b) coextruding lanes of a hydrophobic polymer melt having a top surface onto the upper surface of the polymer web; (c) applying a pressure differential across the screen assembly to form apertures in the continuous layer, wherein the upper surface of said continuous layer is hydrophilic. In a preferred embodiment, the screen assembly has wire-like members in registration with the lanes of hydrophobic polymer. In a further embodiment, the method further comprises forming microstructures on the upper surface of the lanes of hydrophobic polymer.

### BRIEF DESCRIPTION OF DRAWINGS

FIG. 1 shows a plan view of a schematic of an absorbent article that utilizes applicants' apertured film.

FIG. 2 shows a plan view of applicants' apertured film for use as a topsheet in the absorbent article of FIG. 1.

FIG. 3 shows a cross-sectional view of a first embodiment of the absorbent article of FIG. 1 taken along line 2-2 of FIG. 1 wherein the topsheet shown is an enlarged cross-sectional view of the apertured film of FIG. 3 taken along line 4-4 of FIG. 3.

FIG. 4 shows an enlarged cross-sectional view of applicant's apertured film taken along line 5-5 of FIG. 2.

FIG. 5 shows a cross-sectional view of a second embodiment of the absorbent article of FIG. 1 taken along line 2-2 of FIG. 1 wherein the topsheet shown is an enlarged cross-sectional view of the apertured film of FIG. 3 taken along line 4-4 of FIG. 3.

FIG. 6 shows a cross-sectional schematic view of an embodiment of the absorbent article of FIG. 1 taken along line 2-2.

### DETAILED DESCRIPTION

This invention relates to an apertured film for use as a topsheet in an absorbent article. Examples of absorbent articles include diapers, incontinent articles, sanitary napkins, and similar articles.

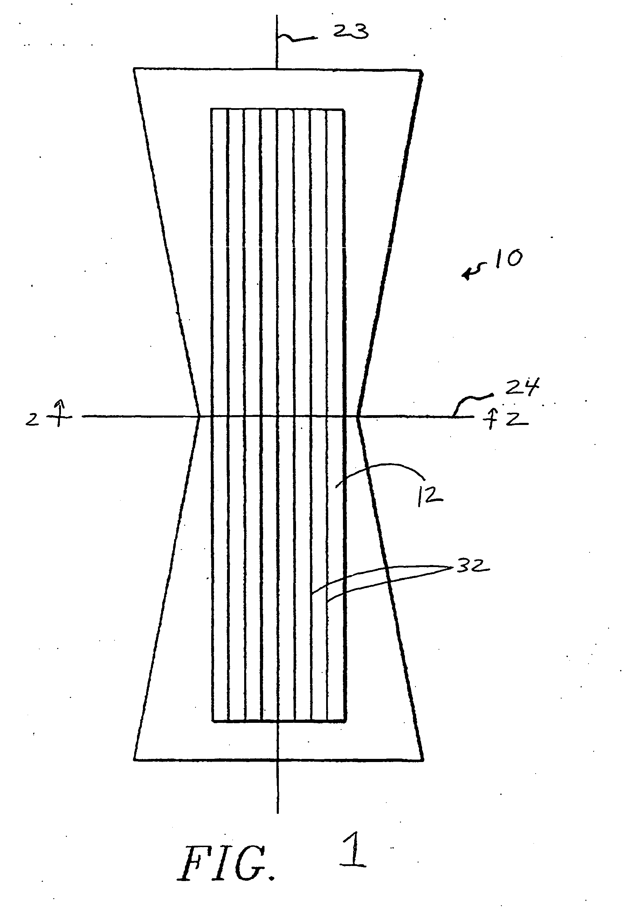

Referring now to FIG. 1, a simplified plan representation of a typical absorbent article 10 is shown. It should be understood, however, that FIG. 1 is shown for purposes of example only, and should not be construed to limit the particular type or configuration of the absorbent article. The absorbent article has two centerlines, a longitudinal centerline 23 and a transverse centerline 24.

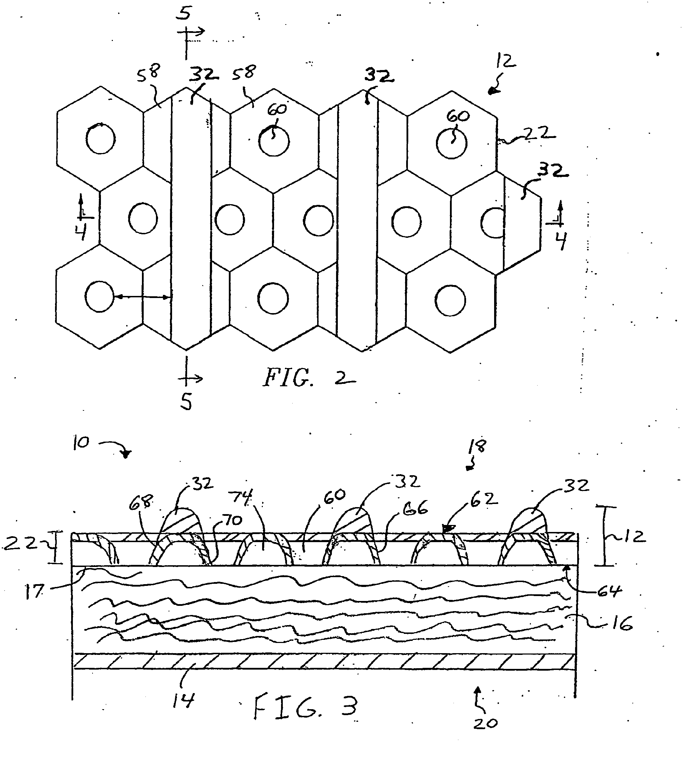

Referring now to FIGS. 2 and 3, an embodiment of the apertured film for use as a topsheet for an absorbent article is shown. The apertured film topsheet comprises an apertured continuous layer 22 and raised lanes of thermoplastic material 32.

As shown in FIG. 3, absorbent article 10 basically comprises topsheet 12, backsheet 14, and an absorbent core 16. Absorbent core 16 has a top or body-facing side 17. Other layers may be included in this general construction.

The absorbent article 10 has two surfaces, a body-contacting surface or body surface 18 and a garment-contacting surface or garment surface 20. The body surface 18 is intended to be worn adjacent to the body of the wearer. The garment surface 20 of the absorbent article 10 is on the opposite side and is intended to be placed adjacent to the wearer's undergarments or clothing when the absorbent article 10 is worn.

The apertured film of the present invention for use as a topsheet of the absorbent article 10 will now be looked at in greater detail. Throughout the remainder of this application, simitar components will share the same numbers for all embodiments of the invention, e.g., "topsheet" will be designated by the numeral 12 in each embodiment.

In a preferred embodiment, the apertured continuous layer 22 is imparted with a repeating pattern. Although a hexagonal pattern is used for purposes of illustration in FIG. 2, it should be understood that other patterns may also be used for any of the films descnbed herein. Examples of other patterns include circular, oval, elliptical, boat-shaped, polygonal, or other suitable patterns or combinations of patterns. The hexagonal pattern forms a plurality of adjacent hexagons or cells 58. In a preferred embodiment, the pattern is based on a mesh count from , 22 to 40 or more preferably from 25 to 32. The mesh count and pattern may be adjusted to improve the tactile impression of the topsheet as disclosed in U.S. Patent No. 6,989,187. Preferably, each cell 58 is provided with an aperture 60, which is large enough to allow insult fluids to be rapidly acquired through the apertured continuous layer 22.

As seen more clearly in FIG. 3, which shows an enlarged cross sectional view of film 12 taken along line 4-4 of FIG. 2, the apertured continuous layer 22 has a male side or garment-facing side 64 and an opposite female side or body-facing side 62. The three dimensional apertured continuous layer 22 has a loft, i.e. the distance between the surface on the female side 62 and the planar surface on the male side 64, of from 250 microns to 625 microns and more preferably 450 microns to 610 microns.

Extending upward from the female side 62 of the apertured continuous layer 22 are raised lanes of thermoplastic material 32. These raised lanes 32 come in contact with the skin of the wearer of the absorbent article 10. The raised lanes 32 preferably run substantially parallel to the longitudinal centerline 23 (FIG. 1) of the absorbent article 10.

As can be seen in FIG. 3, when used as a topsheet for an absorbent article, the topsheet 12 is located adjacent to the top or body-facing side 17 of the absorbent core 16. The apertured continuous layer 22 is a three-dimensional structure having a plurality of drains 66, each of which defines an aperture 60. Each drain has a base opening 68 and an apex opening 70. The apex openings 70 of the drains 66 preferably have a diameter from 250 to 650 microns. The apex openings 70 of the drains 66 are in intimate contact with the absorbent core 16, and preferably apex openings 70 are affixed to core 16 to insure this intimate contact. It should also be noted that essentially only the apex openings 70 of the drains 66 are in intimate contact with the core 16, thereby assuring that the void spaces 74 remain substantially unencumbered. A void volume space 74 is formed on the male side of the apertured continuous layer 22 that provides a fluid passageway between each of the cells 58. Preferably, the ratio of void volume space 74 versus apex opening space 70 is about 2:1.

Preferably, the apertured continuous layer 22 is a perforated thermoplastic film with tapered drains 66 which have a run off percent of less than about 10 percent and which has an increased liquid flow rate through the tapered drains 66. Any thermoplastic material which may be formed into flexible films or sheets may be used in the production of the novel topsheets of the present invention.

Exemplary thermoplastic materials include cellulose esters, nylons and mixed polyamides, polyvinyl alcohol, polyvinyl chloride, polyvinyl acetates, polymethyl methacrylate, polyethylene, polypropylene, blends or copolymer polyolefins, polyesters, polyurethanes, polyethers, polyimides, polyurethanes, polylactic acid, polyacrylic acid, polymethacrylatic acid, polyacrylamide, polyethyleneimine, polyethylene oxide, polystyrene sufonate, and polyethylene glycol and ionomers like SURLYN^{®} which may be formed into flexible film or sheet. Particularly preferred perforated films are polyethylene and polypropylene. One preferred suitable material is a low density polyethylene film. Sheets or film made from such materials may be plasticized with suitable plasticizers and other additives known in the art may be added to achieve the desired physical characteristics.

When using a hydrophobic thermoplastic material such as a polyolefin resin to form the apertured continuous layer 22, the continuous layer must be treated so that at least the female side 62 is hydrophilic. For example, a surfactant can be mixed or blended with the resin prior to the layer being formed from the resin. The surfactant provides a surface which has greater polarity than the polyolefin layer would have without the surfactant being added. Higher surface polarity yields higher wettability.

As used herein, the term "hydrophilic" is used to refer to surfaces that are wettable by aqueous fluids (e.g., aqueous body fluids) deposited thereon. Hydrophilicity and wettability are typically defined in terms of contact angle and the surface tension of the fluids and solid surfaces involved. A surface is said to be wetted by an aqueous fluid (hydrophilic) when the fluid tends to spread spontaneously across the surface. Conversely, a surface is considered to be "hydrophobic" if the aqueous fluid does not tend to spread spontaneously across the surface.

In a preferred embodiment, the surfactant is a non-migrating surfactant. Surfactants typically have a molecular weight of 150 to 300 and contain a hydrophilic end (head) which includes a cationic, anionic or nonionic polar group and a hydrophobic end (tail) usually consisting of one or two aliphatic chains. These surfactants migrate to the film surface where they readily dissolve in aqueous fluid that comes in contact with the film surface. These migrating surfactants lower the surface tension of the fluid to a level less than or equal to that of the film surface energy which results in wetting of the film surface. Because these migrating surfactants dissolve into the aqueous fluid they can be washed from the surface which may change the wetting behavior of the film with multiple insults. Conversely, non-migrating surfactants are usually much larger molecules than typical surfactants and are unable to migrate through the film to any significant degree. The non-migrating surfactants, instead, arrange themselves at the surface of the film to concentrate their polar groups (hydrophilic head) as the top most surface of the film thereby giving the film a much higher surface energy and promoting the wetting by aqueous fluids. Typically, non-migrating surfactants are more permanent and durable over multiple insults. By using a non-migrating surfactant, the surfactant is unlikely to be transferred to the raised lanes 32 when the apertured film 12 is rolled for storage thus preserving the hydrophobicity and dry feel of the raised lanes 32.

Potentially useful non-migrating surfactants may include low molecular weight polyvinyl alcohol, poly acrylic acid, polyethylene oxide, poly methacrylic acid, polyethylene glycol, polyacrylamide, polystyrene sulfonate and ionomers, oligomers of polyethoxy alkanes, and high molecular weight (>450) anionic, cationic, or nonionic surfactants. Particularly preferred non-migrating surfactants are oligomeric ethoxylates such as UNITHOX^{®} 450 from Baker Petrolite.

The raised lanes 32 of the apertured film 12 are preferably made of a hydrophobic thermoplastic material: Exemplary thermoplastic materials include cellulose esters, polyvinyl chloride, polyvinyl acetate, polymethyl methacrylate, polyethylene, polypropylene, blends and copolymers of polyolefins, polyesters, polyimides, and polyurethanes which may be formed into lanes. Particularly preferred thermoplastic materials are polyethylene and polypropylene.

The raised lanes are applied such that the distance from the female side 62 of the apertured continuous layer 22 to the upper surface of the raised lanes 32 is preferably 25 microns to 100 microns. As will be understood by those of skill in the art, the height of the raised lanes will be determined by balancing aesthetic, tactile, and functional considerations. In any event, the raised lanes 32 should be of a height to substantially help prevent the contact of the wearer's skin with the hydrophilic surface of the apertured continuous layer 22. The number of raised lanes 32 and the thickness of the raised lanes 32 is determined so that the wearer's skin does not come in contact with the female side 62 of the apertured continuous layer 22 while still allowing the topsheet 12 to rapidly transmit fluid. Preferably the number of raised lanes 32 and the thickness of the raised lanes 32 is such that the upper surface of the raised lanes 32 cover 5% to 60%, more preferably 10% to 45%, and most preferably 15% to 35%, of the female side 62 of the continuous layer 22.

Alternatively or additionally, a surface treatment can be applied to the upper surface of the raised lanes 32 to make them more hydrophobic. A suitable surface treatment is a silicone release coating or a UV curable silicone. Other suitable treatment materials include, but are not limited to, fluorinated materials such as fluoropolymers (e.g., polytetrafluoroethylene (PTFE), commercially available under the trade name TEFLON®) and chlorofluoropolymers. Other materials which may prove suitable include hydrocarbons such as petrolatum, latexes, paraffins. Silicone materials are presently preferred for use in the absorbent article context for their biocompatibility properties because they have a low affinity for biological materials such as gluco-proteins, blood platelets, and the like. As such, silicone materials tend to resist deposition of biological matter to a greater extent than other materials under in-use conditions. This property enables them to better retain their hydrophobicity as needed for subsequent fluid insults.

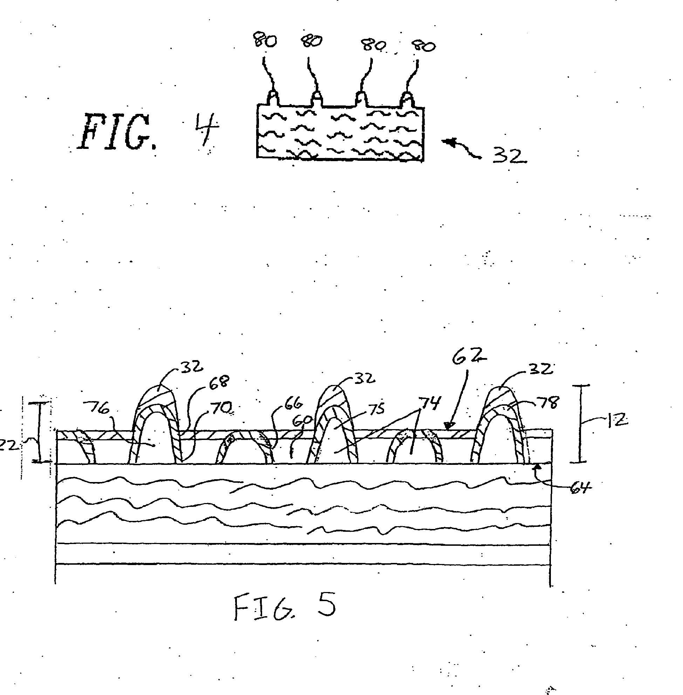

In a preferred embodiment, microstructures may be imparted on the raised lanes 32 to improve the tactile impression of the topsheet 12. Referring to FIG. 4, in a preferred embodiment, microridges 80 are imparted on the raised lanes 32 with a range of height and spacing, and are aligned on a bias, i.e., at an offset angle to a longitudinal centerline 23 (FIG. 1) of the absorbent article 10 for optimum effect. The offset angle may be from 5° to 80°to achieve some effect, but the preferred range is from 30° to 60°, and ideally 45°. The height of the microridges can range from 5 µm to 75 µm, but the preferred range is from : 5 µm to 35 µm. In a particularly preferred embodiment, the microridges have a height of 20 µm. Spacing between microridges can range from 25 µm to 250 µm, but more preferably range from 50 µm to 150 µm. Most preferably, about 95 µm spacing is used.

Referring now to FIG. 5, which shows an enlarged cross sectional view of film 12 taken along line 4-4 of FIG. 2, a second embodiment of the apertured film for use as a topsheet for an absorbent article is shown. From a plan view, the topsheet will look the same as the first embodiment depicted in FIG. 2. Accordingly, as shown in FIG. 2, the topsheet comprises an apertured continuous layer 22 and raised lanes of thermoplastic material 32. In a preferred embodiment, the mesh count and pattern of the apertured continuous layer 22 is as described with respect to FIG. 3 above.

As seen in FIG. 5, the apertured continuous layer 22 has a male side or garment-facing side 64 and an opposite female side or body-facing side 62. Raised ridges extend upward from the female side 62 of the continuous layer 22. In a preferred embodiment, on the upper surface of the raised ridges 78, raised lanes 32 of hydrophobic thermoplastic material are applied.

In a preferred embodiment, the raised lanes 32 are applied to the raised ridges 78 such that the distance from the female side 62 of the apertured continuous layer 22 to the upper surface of the raised lanes 32 is preferably 25 microns to 100 microns. The number, height, and thickness of the raised ridges 78 in conjunction with the raised lanes 32 are the same as discussed above with respect to FIG. 3.

The topsheet 12 of FIG 5. contains drains 66 as discussed above with respect to FIG 3. In the embodiment depicted in FIG. 5, in addition to the void volume space 74, a channel 75 is formed on the male side 64 of each raised ridge 78. Accordingly, an enlarged void volume space 76 is formed when the channel 75 communicates with the void volume space 74 of the apertured plastic film 22.

The thermoplastic materials for use in forming the apertured continuous layer 22 and raised lanes 32 for this embodiment are the same as discussed above. As in the first embodiment, if a hydrophobic thermoplastic material such as a polyolefin resin is used to form the apertured continuous layer 22, then the film must be treated so that at least the female side 62 is hydrophilic. The same methods, as discussed above, may be employed.

Another aspect of the present invention relates to a method for making an apertured film for use as a topsheet for an absorbent article. Accordingly, an embodiment of the invention is a method for making an apertured film for use as a topsheet for an absorbent article, comprising (a) extruding a flat continuous layer of a polymer melt having an upper and lower surface onto a screen assembly; (b) coextruding lanes of a hydrophobic polymer melt having a top surface onto the upper surface of the continuous layer; (c) applying a pressure differential across the screen assembly to form apertures in the continuous layer, wherein the upper surface of the continuous layer is hydrophilic.

In a preferred embodiment a cast coextrusion process is used. In a cast coextrusion process, lanes of a hydrophobic thermoplastic material are extruded through notched zone portals above a flat continuous layer extruded from a slot die. The lanes and flat continuous layer may be extruded from the same die or separate dies used in close proximity to one another. As used herein, the terms "coextrusion" or "coextruding" refers to extruding two or more layers or lanes of thermoplastic material from either a single die or multiple dies used in close proximity to one another.

The lanes and flat continuous layer anneal together as they are subsequently cooled and set by a variety of chilling roller means. In a further embodiment, additional flat continuous layers are extruded between the first continuous layer and the raised lanes, wherein the upper most continuous layer is hydrophilic.

In a preferred embodiment, the topsheet 12 is formed in a direct melt vacuum formed film (VFF) process. In a vacuum formed film process, a pressure differential is applied across a forming screen. In the case of a direct melt VFF process, a molten web is extruded onto a forming area of a forming screen. Alternatively, the web may be reheated and partially melted while the web is over the forming area of the forming screen as taught in U.S. Pat. No. 4,151,240. A melted polymer is desirable to form three-dimensional apertures since a melted polymer is more easily pulled into the apertures in a forming screen. Preferably, the apertures of the forming screen are spaced so as not to create apertures in the raised lanes 32 of the topsheet 12. Both U.S. Pat. Nos. 4,456,570 and 4,151,240 teach primarily using a vacuum to change a two dimensional web into a three-dimensional cell structure and to create apertures in the film. During the formation of a VFF, the polymer of the film typically undergoes a phase change from molten state in a flat form to a crystalline state in the new three dimensional form.

In a preferred embodiment, the screen assembly is able to form the raised ridges 78 (FIG. 5) of the second embodiment. These raised ridges may be formed by, for example, affixing wire-like members to a film contacting surface of an external member, a forming screen, or other film contacting surface. The wire-like members may be round, square, rectangular, elliptical or a polygonal shape. The wire-like members are ideally spaced so that one to five of the underlying openings or cells of the base screen are left unobstructed between adjacent wire-like members. Spacing of the wire-like members should be set to optimize aesthetic, tactile, fluid flow, fluid overflow channeling, and void volume space for fluid distribution and anti-rewet properties of the resulting film products as various applications will dictate. In a preferred embodiment, the number and diameter of the wire-like members correspond to the number and thickness of the raised lanes 32 as discussed above.

The wire-like members may be a continuous wire that is wrapped in a spiral fashion from end to end of the screen assembly with a desired setting of space between the spirals. In a preferred embodiment for use in the present invention, a plurality of individual wires or rings are used and slid over the screen assembly into a desired spatial relationship. Preferably, the wire-like members are spaced so that they are in registration with the raised lanes 32 of the present invention. A variety of metals can be utilized for the wire-like members. For example, nickel, copper, aluminum, stainless steel, carbon steel, brass, bronze, and others are appropriate. It is preferable to match the thermal expansion of the wire metal with that of the base screen.

In addition to metal wires, wires of other materials may be used. An example of another material that may be used is a high temperature engineering polymer material such as polyamide-imide, polyester, polyetheretherketone, polyacetal, polyetherimide, polyethersulfone, polyphenylenesulfide, or polytetrafluoroethylene. Regardless of the material selected for the wire-like member, it is necessary that its softening temperature exceeds the temperature of the molten resin being extruded onto the screen assembly.

In a further embodiment, the method further comprises forming microstructures on the upper surface of the lanes of hydrophobic polymer. Any known method for imparting such microstructures may be used including the use of a microtextured screen or by cloth embossing.

In practice, the apertured film 12 may be used as a topsheet in an absorbent article 10. Absorbent article 10 is used for applications where fluid absorption is desirable. In use, body exudates, such as urine, are deposited on the absorbent article 10. As the urine contacts the hydrophobic raised lanes 32, it will run-off the lanes and be drawn toward the hydrophilic surface of the apertured continuous layer and through the topsheet to the absorbent core 16. Any residual fluid that remains on the female side or body side 62 of the apertured continuous layer 22 is unlikely to come in contact with the wearer's skin because the raised lanes 32 substantially help prevent the skin from contacting the apertured continuous layer 22. As an additional benefit, the space created between the wearer's skin and the body side 62 of the apertured continuous layer allows air to flow which can aid in the drying of the topsheet 12.

As each urine insult is typically delivered to the same spot on the absorbent article, upon repeated insults, an undesirable leakage or undesirable feeling of wetness by the wearer may occur due to the core material 16 becoming saturated in the repeat insult region. In other words, the absorbent core 16 may experience an inability to absorb repeated insults in a particular region. As a result, additional fluid insults that are delivered to the absorbent article 10 may be unabsorbed by the core 16 and remain on the upper or body-facing side 17 of the core layer 16. In addition to providing a dry top, applicant's topsheet 12 provides a way for the unabsorbed fluid from the core layer 16 to be directed to unsaturated zones of the core layer 16.

For example, when the topsheet 12 of FIG. 3 is used in absorbent article 10 (FIG. 1), fluid that is not absorbed or that spills-over from core layer 16 is able to flow within void volume space 74 to an unsaturated area of core 16. Likewise, when the topsheet 12 of FIG. 5 is used in an absorbent article 10 (FIG. 1), fluid that is not absorbed or that spills-over from core layer 16 is able to flow within both the void volume spaces 74 and 76 to an unsaturated area of core 16. As an additional advantage of the present invention, if the void volume space is filled, the fluid that is not absorbed is able to flow between the raised lanes 32 over the female side 62 of the apertured continuous layer 22 to an unsaturated area of core 16.

A further advantage of the raised lanes 32, independent of or in conjunction with the raised ridges 78, is that the raised lanes 32 direct unabsorbed fluids in a desired direction, such as in the longitudinal direction, i.e., parallel to longitudinal centerline 23 of disposable diaper 10 (FIG. 1). By directing the unabsorbed fluid in the longitudinal direction, the fluid may be directed to locations with greater amounts of unsaturated core material 16 as opposed to directing the fluid toward undesirable locations such as a perimeter of the diaper. The raised lanes 32 direct fluid away from a direction that is parallel to the transverse centerline 24 of absorbent article 10. The raised lanes 32 are, therefore, effective at eliminating side leakage from disposable diaper 10.

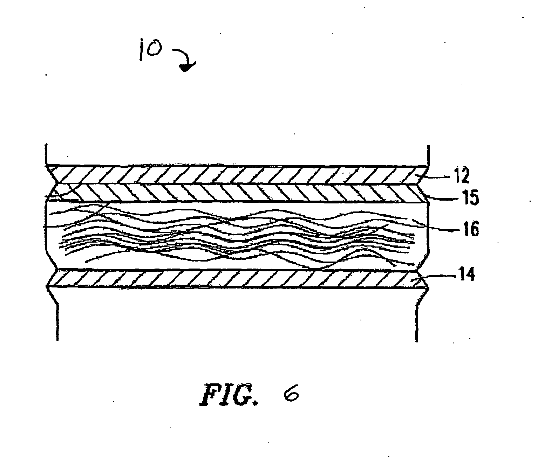

Additionally, various embodiments of applicant's topsheet 12 can be combined with acquisition distribution layers to provide a further enlarged void volume space to accommodate unabsorbed fluids. Referring to FIG. 6, which shows a cross-sectional schematic view of an embodiment of the absorbent article of FIG. 1 taken along line 2-2, the absorbent article 10 basically comprises topsheet 12, backsheet 14, and an absorbent core 16, and an acquisition distribution layer 15 between the topsheet 12 and the absorbent core 16. The further enlarged void volume space of the acquisition distribution layer 15 allows unabsorbed fluids to flow to regions where core material 16 is unsaturated without allowing the unabsorbed fluids to come into contact with the topsheet 12, thereby avoiding the risk of wetness to the wearer.

## Claims

1. An apertured film for use as a topsheet for an absorbent article comprising:
an apertured continuous layer having a male side and an opposite female side, said female side having a surface that is hydrophilic; and
raised lanes extending upward from said female side having an upper surface, said upper surface of said raised lanes being hydrophobic.

2. The apertured film of claim 1 wherein said raised lanes are oriented substantially parallel to one another.

3. The apertured film of claim 1 wherein said apertured continuous layer has raised ridges extending upward from said female side in registration with said raised lanes.

4. The apertured film of claim 1 wherein said upper surface of said raised lanes extend from 25 microns to 100 microns above said female side of said continuous layer.

5. The apertured film of claim 1 wherein said raised lanes cover 5 percent to 60 percent of said female side of said continuous layer.

6. The apertured film of claim 1 wherein said continuous layer contains a surfactant.

7. The apertured film of claim 6 wherein said surfactant is non-migrating.

8. The apertured film of claim 7 wherein said non-migrating surfactant is an oligomeric ethoxylate

9. The apertured film of claim 1 wherein said raised lanes are a low density polyethylene.

10. The apertured film of claim 1 wherein said upper surface of said raised lanes contain microstructures.

11. An absorbent article comprising a topsheet formed of the apertured film of claim 1, a backsheet, and an absorbent core between said topsheet and said backsheet.

12. The absorbent article of claim 11 further comprising an acquisition distribution layer between said topsheet and said absorbent core.

13. A method of making an apertured film for use as a topsheet for an absorbent article, comprising:
extruding a flat continuous layer of a polymer melt having an upper and lower surface onto a screen assembly, wherein said upper surface of said continuous layer is hydrophilic;
coextruding lanes of a hydrophobic polymer melt having an upper surface onto said upper surface of said continuous layer, and
applying a pressure differential across said screen assembly to form apertures in said continuous layer.

14. The method of claim 13 wherein said screen assembly has wire-like members in registration with said lanes of hydrophobic polymer.

15. The method of claim 13 wherein said raised lanes extend from 25 microns to 100 microns above said upper surface of said continuous layer.

16. The method of claim 13 wherein said raised lanescover 5 percent to 60 percent of said upper surface of said continuous layer.

17. The method of claim 13 wherein said continuous layer contains a non-migrating surfactant.

18. The method of claim 17 wherein said non-migrating surfactant is an
oligomeric ethoxylate.

19. The method of claim 13 further comprising:
forming microstructures on said upper surface of said lanes of hydrophobic polymer.

20. The method of claim 13, wherein prior to extruding the lanes of hydrophobic polymer melt, one or more additional flat continuous layers of polymer melt are extruded onto said upper surface of said first continuous layer, wherein the uppermost layer is hydrophilic.

## Patentansprüche

1. Perforierte Folie zur Verwendung als Innenvlies für einen saugfähigen Artikel, umfassend:
eine perforierte kontinuierliche Schicht mit einer männlichen Seite und einer gegenüberliegenden weiblichen Seite, wobei die weibliche Seite eine hydrophile Oberfläche aufweist; und
erhabene Bahnen, die sich von der weiblichen Seite ausgehend nach oben erstrecken und eine obere Fläche aufweisen, wobei die obere Fläche der erhabenen Bahnen hydrophob ist.

2. Perforierte Folie gemäß Anspruch 1, wobei die erhabenen Bahnen im Wesentlichen parallel zueinander ausgerichtet sind.

3. Perforierte Folie gemäß Anspruch 1, wobei die perforierte kontinuierliche Schicht erhabene Grate, die sich von der weiblichen Seite ausgehend nach oben erstrecken, in Registerausrichtung mit den erhabenen Bahnen aufweist.

4. Perforierte Folie gemäß Anspruch 1, wobei sich die obere Fläche der erhabenen Bahnen 25 µm bis 100 µm oberhalb der weiblichen Seite der kontinuierlichen Schicht erstreckt.

5. Perforierte Folie gemäß Anspruch 1, wobei die erhabenen Bahnen 5 Prozent bis 60 Prozent der weiblichen Seite der kontinuierlichen Schicht bedecken.

6. Perforierte Folie gemäß Anspruch 1, wobei die kontinuierliche Schicht ein Tensid enthält.

7. Perforierte Folie gemäß Anspruch 6, wobei das Tensid nichtmigrierend ist.

8. Perforierte Folie gemäß Anspruch 7, wobei das nichtmigrierende Tensid ein oligomeres Ethoxylat ist.

9. Perforierte Folie gemäß Anspruch 1, wobei die erhabenen Bahnen aus einem Polyethylen geringer Dichte bestehen.

10. Perforierte Folie gemäß Anspruch 1, wobei die obere Fläche der erhabenen Bahnen Mikrostrukturen enthält.

11. Saugfähiger Artikel, das ein Innenvlies, das aus der perforierten Folie gemäß Anspruch 1 besteht, eine Außenfolie und einen Saugkern zwischen dem Innenvlies und der Außenfolie umfasst.

12. Saugfähiger Artikel gemäß Anspruch 11, der weiterhin eine Aufnahme/Verteilung-Schicht zwischen dem Innenvlies und dem Saugkern umfasst.

13. Verfahren zur Herstellung einer perforierten Folie zur Verwendung als Innenvlies für einen saugfähigen Artikel, umfassend:
Extrudieren einer flachen kontinuierlichen Schicht aus einer Polymerschmelze mit einer oberen und einer unteren Fläche auf eine Siebgruppe, wobei die obere Fläche der kontinuierlichen Schicht hydrophil ist;
Coextrudieren von Bahnen aus einer hydrophoben Polymerschmelze mit einer oberen Fläche auf die obere Fläche der kontinuierlichen Schicht; und
Anwenden eines Druckgefälles über die Siebgruppe unter Bildung von Öffnungen in der kontinuierlichen Schicht.

14. Verfahren gemäß Anspruch 13, wobei die Siebgruppe drahtartige Elemente in Registerausrichtung mit den Bahnen aus hydrophobem Polymer aufweist.

15. Verfahren gemäß Anspruch 13, wobei sich die erhabenen Bahnen 25 µm bis 100 µm oberhalb der oberen Fläche der kontinuierlichen Schicht erstrecken.

16. Verfahren gemäß Anspruch 13, wobei die erhabenen Bahnen 5 Prozent bis 60 Prozent der oberen Fläche der kontinuierlichen Schicht bedecken.

17. Verfahren gemäß Anspruch 13, wobei die kontinuierliche Schicht ein nichtmigrierendes Tensid enthält.

18. Verfahren gemäß Anspruch 17, wobei das nichtmigrierende Tensid ein oligomeres Ethoxylat ist.

19. Verfahren gemäß Anspruch 13, weiterhin umfassend:
Bilden von Mikrostrukturen auf der oberen Fläche der Bahnen aus hydrophobem Polymer.

20. Verfahren gemäß Anspruch 13, wobei vor dem Extrudieren der Bahnen aus der hydrophoben Polymerschmelze eine oder mehrere zusätzliche flache kontinuierliche Schichten aus Polymerschmelze auf die obere Fläche der ersten kontinuierlichen Schicht extrudiert werden, wobei die oberste Schicht hydrophil ist.

## Revendications

1. Film perforé destiné à être utilisé en tant que feuille supérieure pour un article absorbant comprenant :
une couche continue perforée ayant un côté mâle et un côté opposé femelle, ledit côté femelle ayant une surface qui est hydrophile ; et
des bandes surélevées s'étendant vers le haut à partir dudit côté femelle ayant une surface supérieure, ladite surface supérieure desdites bandes surélevées étant hydrophobe.

2. Film perforé selon la revendication 1, dans lequel lesdites bandes surélevées sont orientées essentiellement de manière parallèle les unes aux autres.

3. Film perforé selon la revendication 1, dans lequel ladite couche continue perforée comporte des stries surélevées s'étendant vers le haut à partir dudit côté femelle coïncidant avec lesdites bandes surélevées.

4. Film perforé selon la revendication 1, dans lequel ladite surface supérieure desdites bandes surélevées dépasse de 25 microns à 100 microns au-dessus dudit côté femelle de ladite couche continue.

5. Film perforé selon la revendication 1, dans lequel lesdites bandes surélevées recouvrent 5 pour cent à 60 pour cent dudit côté femelle de ladite couche continue.

6. Film perforé selon la revendication 1, dans lequel ladite couche continue contient un tensioactif.

7. Film perforé selon la revendication 6, dans lequel ledit tensioactif ne migre pas.

8. Film perforé selon la revendication 7, dans lequel le tensioactif qui ne migre pas est un éthoxylate oligomérique.

9. Film perforé selon la revendication 1, dans lequel lesdites bandes surélevées sont en polyéthylène basse densité.

10. Film perforé selon la revendication 1, dans lequel ladite surface supérieure desdites bandes surélevées contient des microstructures.

11. Article absorbant comprenant une feuille supérieure formée par le film perforé de la revendication 1, une feuille arrière et un coeur absorbant entre ladite feuille supérieure et ladite feuille arrière.

12. Article absorbant selon la revendication 11 comprenant en outre une couche de réception et de diffusion entre ladite feuille supérieure et ledit coeur absorbant.

13. Procédé de fabrication d'un film perforé destiné à être utilisé en tant que feuille supérieure pour un article absorbant comprenant :
l'extrusion d'une couche continue plate d'un polymère fondu ayant une surface supérieure et inférieure sur un ensemble à tamis, ladite surface supérieure de ladite couche continue étant hydrophile ;
la coextrusion de bandes d'un polymère fondu hydrophobe ayant une surface supérieure sur ladite surface supérieure de ladite couche continue ; et
l'application d'un différentiel de pression sur ledit ensemble à tamis pour former des ouvertures dans ladite couche continue.

14. Procédé selon la revendication 13, dans lequel ledit ensemble à tamis comporte des éléments de type fil coïncidant avec lesdites bandes de polymère hydrophobe.

15. Procédé selon la revendication 13, dans lequel lesdites bandes surélevées dépassent de 25 microns à 100 microns au-dessus de ladite surface supérieure de ladite couche continue.

16. Procédé selon la revendication 13, dans lequel lesdites bandes surélevées recouvrent 5 pour cent à 60 pour cent de ladite surface supérieure de ladite couche continue.

17. Procédé selon la revendication 13, dans lequel ladite couche continue contient un tensioactif qui ne migre pas.

18. Procédé selon la revendication 17, dans lequel le tensioactif qui ne migre pas est un éthoxylate oligomérique.

19. Procédé selon la revendication 13 comprenant en outre la formation de microstructures sur ladite surface supérieure desdites bandes de polymère hydrophobe.

20. Procédé selon la revendication 13, dans lequel avant l'extrusion des bandes de polymère fondu hydrophobe, une ou plusieurs couches continues plates supplémentaires de polymère fondu sont extrudées sur ladite surface supérieure de ladite première couche continue, dans lequel la couche la plus haute est hydrophile.
